(19) Europäisches Patentamt
European Patent Office
Office européen des brevets

(11) Veröffentlichungsnummer: **0 252 358 B1**

# (12) EUROPÄISCHE PATENTSCHRIFT

(45) Veröffentlichungstag der Patentschrift: **06.03.91**

(51) Int. Cl.5: **C07D 303/24**, C09K 19/34, C08G 59/24, C08G 59/38

(21) Anmeldenummer: **87108933.0**

(22) Anmeldetag: **23.06.87**

## (54) Flüssig-kristalline Diglycidylverbindungen, ihre Herstellung und Verwendung in härtbaren Epoxid-Gemischen.

(30) Priorität: **05.07.86 DE 3622610**

(43) Veröffentlichungstag der Anmeldung: **13.01.88 Patentblatt 88/02**

(45) Bekanntmachung des Hinweises auf die Patenterteilung: **06.03.91 Patentblatt 91/10**

(84) Benannte Vertragsstaaten: **AT DE FR GB IT**

(56) Entgegenhaltungen:

**CHEMICAL ABSTRACTS, Band 100, Nr. 22, 28.05.1984, Columbus, Ohio, USA YURECHKO, N A ; SOROKINA A N ; KLEBANOV M S ; SHOLOGON I M ; "Polymeric composition for producing reinforced plastics", Seite 44, Spalte 2, Z'fassung 175 935u**

**CHEMICAL ABSTRACTS, Band 86, Nr. 22, 30.05.1977, Columbus, Ohio, USA NAKAMURA K ; SASAGURI K ; MATSUMOTO Y ; SATO M ; MATSUO S ; "Thermosetting powdered resin compositions", Seite 88, Spalte 1, Z'fassung 157 145z**

(73) Patentinhaber: **BAYER AG**
**W-5090 Leverkusen 1 Bayerwerk(DE)**

(72) Erfinder: **Müller, Hanns-Peter, Dr.**
**Im Kerberich 6**
**W-5068 Odenthal(DE)**
Erfinder: **Gipp, Roland, Dr.**
**Jakob-Böhme-Strasse 2**
**W-5000 Köln 80(DE)**
Erfinder: **Heine, Heinrich, Dipl.-Ing.**
**Müritzstrasse 38**
**W-5090 Leverkusen 1(DE)**

**EP 0 252 358 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue, flüssig-kristalline Diglycidylverbindungen von gegebenenfalls ringsubstituierten 4-Hydroxyphenyl-4-hydroxybenzoaten, eine Synthese von p-Epoxypropoxy-phenyl-p-epoxy-propoxybenzoat, ferner Epoxidharzgemische, enthaltend die neuen Diglycidylverbindungen sowie die Verwendung der neuen Diglycidylverbindungen und deren Epoxidharzgemische in härtbaren Mischungen.

Unter den technisch gebräuchlichen Epoxidharzen kommt den Diglycidylethern des "Bisphenol-A" die größte Bedeutung zu. Die Eigenschaften der ausgehärteten Harze werden sehr stark von der Struktur der Reaktionspartner, also der Diglycidylether und der Art des Härters beeinflußt.

Diglycidylverbindungen von gegebenenfalls ringsubstituierten 4-Hydroxy-phenyl-4-hydroxybenzoaten sind bisher nicht bekannt geworden.

Es hat sich gezeigt, daß diese Verbindungen flüssigkristallinen Charakter besitzen und damit den Aufbau von Epoxidharzen mit besonderen physikalischen Eigenschaften ermöglichen.

Es wurde überraschenderweise auch gefunden, daß sich diese Diglycidylverbindungen von gegebenenfalls ringsubstituierten 4-Hydroxyphenyl-4-hydroxy-benzoaten in guten Ausbeuten gewinnen lassen, wenn man die Synthese in Gegenwart von Natriumhydroxid mit überschüssigem Epichlorhydrin in an sich bekannter Weise durchführt. Diese Tatsache ist deswegen überraschend, da der Fachmann erwarten mußte, daß unter den angewendeten Reaktionsbedingungen Arylbenzoate hydrolytisch gespalten werden. Dies ist überraschenderweise praktisch nicht, d.h. in nur sehr untergeordnetem Maße, der Fall.

Gegenstand der Erfindung sind somit Diglycidylverbindungen der Formel (I)

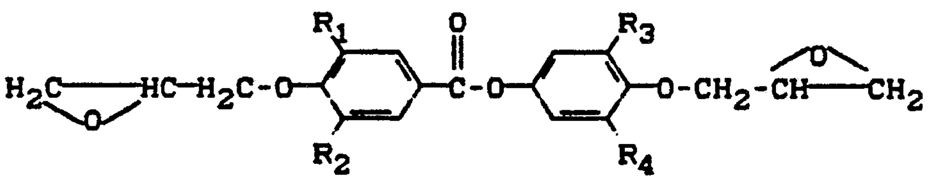

(I)

worin

$R_1,R_2,R_3,R_4$ unabhängig voneinander Wasserstoff, Halogen oder Alkyl mit 1 bis 6 C-Atomen, vorzugsweise 1 bis 4 C-Atomen bedeuten.

Gegenstand der Erfindung ist auch ein Verfahren zur Herstellung der Diglycidylverbindingen der Formel (I), dadurch gekennzeichnet, daß man gegebenenfalls ringsubstituierte 4-Hydroxyphenyl-4-hydroxybenzoate (II) mit überschüssigem Epichlorhydrin in Gegenwart von Katalysatoren und Alkaliverbindungen in an sich bekannter Weise umsetzt.

Gegenstand der vorliegenden Erfindung sind schließlich (härtbare) Mischungen, enthaltend Diglycidylverbindungen der Formel (I), gegebenenfalls in Abmischung mit an sich bekannten Di-, Tri- und Tetraglycidylverbindungen (und Härtungsmitteln für Epoxidharze).

Die Mischungen enthalten vorzugsweise mindestens 5 Gew.-% (I), vorzugsweise mindestens 20 Gew.-% (I).

Die erfindungsgemäßen Verbindungen der Formel (I) können hergestellt werden, indem man ein Bisphenol der Formel (II)

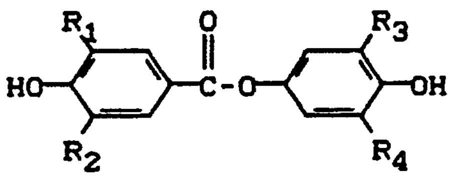

(II)

worin

$R_1,R_2,R_3,R_4$ die gleiche Bedeutung wie in Formel (I) haben,

mittels eines Epihalogenhydrins zu einer Verbinbundung der Formel (I) glycidyliert.

Die Glycidylierung von Hydroxylgruppen aufweisenden organischen Verbindungen stellt eine grundsätzlich bekannte Umsetzungsform dar. Bisphenole der Formel (II) können beispielsweise mit Epichlorhydrin zu einem Dichlorhydrin der Formel (III) umgesetzt werden, das dann dehydrochloriert wird.

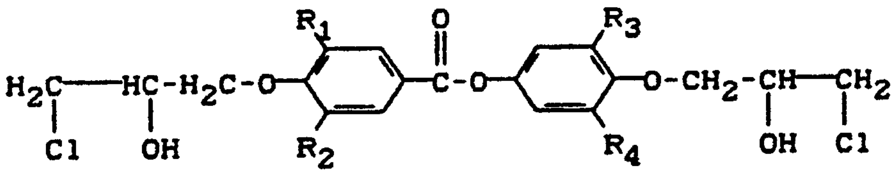

(III)

Man arbeitet vorzugsweise nach folgender Methode: In einem 2-Stufenverfahren wird zuerst 1 Mol Bisphenol mit wenigstens 10 Mol, vorzugsweise 20 bis 40 Mol Epichlorhydrin in Gegenwart eines Katalysators (z.B. Tetraalkylammoniumchlorid) behandelt, wobei ein Dichlorhydrin der Formel (III) entsteht. Im Gleichgewicht bilden sich schon während der Reaktion Glycidether und 1,3-Dichlorisopropanol durch Umsetzung von 3-Chlor-2-hydroxypropylethergruppen mit Epichlorhydrin. In der zweiten Stufe wird der Bischlorhydrinether (III) mit Alkali behandelt, wobei die Epoxidgruppen gebildet werden und das vorhandene 1,3-Dichlorisopropanol in Epichlorhydrin rücküberführt wird.

Das Alkali ist normalerweise Natriumhydroxid, doch können auch andere alkalische Substanzen wie Bariumhydroxid oder Kaliumcarbonat für die Umwandlung der 1,2-Chlorhydringruppen in die 1,2-Epoxidgruppen verwendet werden.

Die Umsetzung kann auch in einem Lösungsmittel, beispielsweise Kohlenwasserstoffen, Ethern oder Ketonen, durchgeführt werden, doch wird vorzugsweise ein Überschuß an Epichlorhydrin als Lösungsmittel verwendet. Die Umsetzung wird allgemein bei erhöhter Temperatur, etwa bei 40 bis 100 ° C, durchgeführt.

Vorzugsweise verwendet man als Verbindungen der Formel (II) solche, worin $R_1$, $R_2$, $R_3$, $R_4$ unabhängig voneinander Wasserstoff, Chlor, Brom oder Alkyl mit 1 bis 4 C-Atomen bedeuten; besonders bevorzugt wird die Verbindung I mit $R_1$, $R_2$, $R_3$, $R_4$ = Wasserstoff.

Die Synthese des Bisphenols der Formel (II), worin $R_1$, $R_2$, $R_3$, $R_4$ je ein Wasserstoffatom bedeuten, ist in der gleichzeitig eingereichten deutschen Patentanmeldung P 36 22 611 beansprucht.

Die erfindungsgemäßen Epoxidharzgemische mit an sich bekannten Di-, Tri- und Tetraglycidylverbindungen werden vorzugsweise durch einfaches Mischen einer Diglycidylverbindung der Formel (I) mit den bekannten Epoxidharzen, wie sie z.B. in Polymere Werkstoffe, Band III, Technologie 2, herausgegeben von H. Batzer, Seite 171 bis 174, Georg Thieme Verlag, Stuttgart/New York, 1984 beschrieben sind, hergestellt.

Eine andere Möglichkeit zur Herstellung der erfindungsgemäßen Epoxidharzgemische besteht in der Glycidylierung eines Gemisches aus Bisphenolen der Formel (II) und z.B. Bisphenol A, aromatischen Diaminen, Aminophenolen oder Heterocyclen wie z.B. Cyanursäure.

Die erfindungsgemäßen Diglycidylverbindungen und deren Gemische mit bekannten Epoxidharzen können mit den üblichen Härtungsmitteln für Epoxidharze ausgehärtet werden.

Als Beispiele für Härtungsmittl seien die gebräuchlichen Härtungsmittel für Epoxidharze genannt, einschließlich der aliphatischen, cycloaliphatischen, aromatischen und heterocylischen Amine, wie Bis-(4-aminophenyl)-methan, Anilin-Formaldehyd-Harz, Bis-(4-aminophenyl)-sulfon, Propan-1,3-diamin, Hexamethylendiamin, Diethylentriamin, Triethylentetramin, 2,2,4-Trimethylhexan-1,6-diamin, m-Xylylendiamin, Bis-(4-aminocyclohexyl)-methan, 2,2-Bis-(4-aminocyclohexyl)-propan und 3-Aminomethyl-3,5,5-trimethylcyclohexylamin (Isophorondiamin), Polyaminoamide, wie beispielsweise solche aus aliphatischen Polyaminen und dimerisierten oder trimerisierten Fettsäuren, Polyphenole, wie Resorcin, Hydrochinon, 2,2-Bis-(4-hydroxyphenyl)-propan und Phenol-Aldehyd-Harze, Polythiol, wie die im Handel unter der Bezeichnung "Thiokole" erhältlichen Polythiole, Polycarbonsäuren und ihre Anhydride, wie z.B. Phthalsäureanhydrid, Tetrahydrophthalsäureanhydrid, Hexahydrophthalsäureanhydrid,
Hexachlorendomethylentetrahydrophthalsäureanhydrid,
Pyromellitsäureanhydrid, Benzophenon-3,3',4,4'-tetracarbonsäuredianhydrid, die Säuren der zuvorgenannnten Anhydride sowie auch Isophthalsäure und Terphthalsäure. Es können auch katalytisch wirkende Härtungsmittel verwendet werden, die beispielsweise tertiäre Amine [z.B. 2,4,6-Tris-(dimethylaminoethyl)-phenol)], Imidazole und andere Mannichbasen; Alkalimetallalkoxide von Alkoholen (z.B. Na-Alkoholat von 2,4-Dihydroxy-3-hydroxymethylpentan), Zinnsalze von Alkansäuren (z.B. Zinnoctanoat), Friedel-Crafts-Katalysatoren, wie Bortrifluorid und Bortrichlorid und ihre Komplexe und Chelate, die durch Umsetzung von Bortifluorid mit z.B. 1,3-Diketonen erhalten werden.

Mit den Härtungsmitteln können auch die geeigneten Härtungsbeschleuniger eingesetzt werden. Bei Verwendung von Poly-(aminoamiden), Polythiole oder Polycarbonsäureanhydriden können tertiäre Amine oder deren Salze, quaternäre Ammoniumverbindungen oder Alkalimetallalkoxide als Beschleuniger dienen.

Die Menge des eingesetzten Härtungsmittels richtet sich nach der chemischen Natur des Härtungsmittels und nach den gewünschten Eigenschaften der härtbaren Mischung und des gehärteten Produktes. Die maximale Menge kann leicht ermittelt werden. Wenn das Härtungsmittel ein Amin ist, werden normalerweise 0,75 bis 1,25 Äquivalente Aminwasserstoff pro 1 Epoxidäquivalent eingesetzt. Wenn Polycarbonsäuren oder ihre Anhydride eingesetzt werden, verwendet man gewöhnlich 0,4 bis 1,1 Äquivalente Carboxylgruppe bzw. Anhydridgruppe per 1 Äquivalent Epoxidgruppe. Bei der Verwendung von Polyphenolen als Härtungsmittel setzt man zweckmäßig 0,75 bis 1,25 phenolische Hydroxylgruppen per 1 Epoxidäquivalent ein.

Katalytisch wirkende Härtungsmittel werden allgemein in Mengen von 1 bis 40 Gew.-Teilen pro 100 Gew.-Teile Epoxidharz eingesetzt.

Je nach Natur des verwendeten Härtungsmittels kann die Härtung bei Raumtemperatur oder bei höheren Tempraturen vorgenommen werden. Die Härtung kann gewünschtenfalls auch in zwei Stufen vorgenommen werden, indem man z.B. den Härtungsvorgang unterbricht oder, falls man ein Härtungsmittel für höhere Temperaturen einsetzt, die härtbare Mischung bei tieferen Temperaturen teilweise härten läßt. Die dabei erhaltenen Produkte sind noch schmelzbare und lösliche Präkondensate (sogenannte "B-Stufenharze") und eignen sich z.B. für Pressmassen, Sinterpulver oder Prepregs.

Die erfindungsgemäßen härtbaren Mischungen können ferner noch Plastifizierungsmittel, wie Dibutylphthalat, Dioctylphthalat oder Trikresylphosphat, oder Additive erhalten, wie Streckmittel, Füllstoffe, Verstärkungsmittel, Färbemittel, Fließmittel und Formtrennmittel. Geeignete Streckmittel, Füllstoffe und Verstärkungsmittel sind beispielsweise Asbest, Asphalt, Bitumen, Glasfasern, Textilfasern, Kohlenstoff- oder Borfasern, Glimmer, Tonerde, Gips, Titandioxid, Kreide, Quarzmehl, Cellulose, Kaolin, gemahlener Dolomit, Wollastonit, Kieselerde mit großer spezifischer Oberfläche (erhältlich unter dem Handelsnamen "Aerosil®"), mit langkettigen Aminen modifizierte Tonerde (erhältlich unter dem Handelsnamen "Bentone®"), gepulvertes Polyvinylchlorid, Polyolefin oder Aminoplaste, Metallpulver, wie Aluminium-oder Eisenpulver. Flammschutzmittel, wie Antimontrioxid, können ebenfalls den härtbaren Mischungen zugegeben werden.

Die erfindungsgemäßen härtbaren Massen lassen sich z.B. als Laminier-, Tränk- und Gießharze, Pulverbeschichtungen, Formmassen, Kitte und Dichtungsmassen, Einbettungs-und Isoliermassen für die Elektrotechnik und insbesondere als Klebstoffe oder Matrixharze zur Herstellung von faserverstärkten Kunststoffen verwenden.

## Beispiele

### Beispiel 1

Herstellung von p-Epoxy-propoxyphenyl-p-epoxypropoxybenzoat (I)

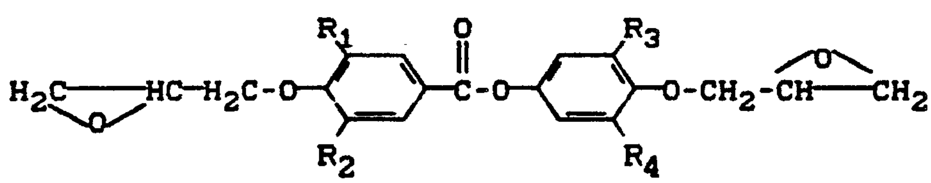

**Ansatz:** 2275 g (30 Mol) Epichlorhydrin
4 g Tetraethylammoniumchlorid
230 g (1 Mol) 4-Hydroxyphenyl-4-hydroxybenzoat (II)
222 g (2,5 Mol) 45 %ige NaOH

In einem 4 Ltr. 3-Hale-Kolben, der mit Innenthermometer, Rührer und - über einen Wasserabscheider - einem Rückflußkühlar versehen ist, wird das Epichlorhydrin, das 4-Hydroxyphenyl-4-hydroxybenzoat (II) und das Tetraethylammoniumchlorid 16 Stunden bei 60 °C unter $N_2$ zur Reaktion gebracht. Die anschließende Dehydrohalogenierung erfolgt innerhalb von 6 Stunden bei 130 mbar und 60 °C durch kontinuierliches Zutropfen der wäßrigen NaOH. Das Wasser wird dabei kontinuierlich über den Wasserabscheider ausgekreist.

Nach erfolgter NaOH-Zugabe wird noch 3 Stunden bei den gleichen Bedingungen nachgerührt.

Anschließend wird das Kochsalz abfiltriert, mit frischem Epichlorhydrin gewaschen und die vereinigten Filtrate bei 50° C Badtemperatur und 13 mbar eingeengt. Das abdestillierte Epichlorhydrin wird für den nächsten Ansatz wiederverwendet. Der Ruckstand wird in 200 ml Acetonitril gelöst und nach Zugabe von 1 l Methanol zur Kristallisation gebracht. Die Kristalle werden abgesaugt, mit Methanol gewaschen und bei Raumtemperatur unter Vakuum über Silikagel getrocknet.

Ausbeute: 242 g (71 % der Theorie)

Fp.: 105-107° C. Nach zweimaligem Umkristallisieren aus Acetonitril/Isopropanol (1:1) steigt der Fp. auf 118° C an.

Fp.: 118° C (Differential Termo-Analyse)

p-Epoxypropoxyphenyl-p-epoxypropoxybenzoat besitzt flüssig-kristallinen Charakter:

Quantitative DTA (Differenzialthermoanalyse) und polarisationsmikroskopische Messungen zeigen, daß die Verbindung beim Abkühlen aus der Schmelze bei 93° C in den nematischen Zustand und bei 80° C in den festen Zustand übergeht.

DTA-Messung, Gerät Mettler TA 2000, Quantitative Meßzellen, Alu-Tiegel, Übersichtsmessung: 2 K/min.

Polarisationsmikroskopische Messung: Gerät Mettler FP 6, 60-fache Vergrößerung, gekreuzte Polarisationsfilter.

Spezielle Härtungsreaktionen unter Ausnutzung des flüssig-kristallinen Verhaltens des p-Epoxypropoxyphenyl-p-apoxy-propoxybenzoats (I) sind in der deutschen Patentanmeldung p (Le A 24 624) beschrieben.

Beispiel 2

100 Gew.-Teile des p-Epoxypropoxyphenyl-p-epoxypropoxybenzoats (I) aus Beispiel 1 werden mit 90 Gew.-Teilen Hexahydrophthalsäureanhydrid und 1,9 Gew.-Teilen Dimethylbenzylamin auf 80° C erwärmt. Dabei entsteht ein flüssiges, homogenes Gemisch. Das Gemisch wird in eine Form gegossen (200 mm x 300 mm x 4 mm) 4 Stunden bei 80° C, danach 16 Stunden bei 120° C gehärtet. Aus dem erhaltenen Formkörper werden Prüfkörper geschnitten und deren mechanische Eigenschaften ermittelt.

| | |
|---|---|
| Zugfestigkeit (MPa) | 65 |
| Reißdehnung (%) | 3,2 |
| E-Modul aus Zugversuch (MPa) | 3070 |
| Biegefestigkeit (MPa) | 122 |
| Randfaserdehnung (%) | 5,7 |
| Schlagzähigkeit (kJ/m²) | 20,5 |
| Kugeldruckhärte (MPa) | 164 |
| Martensgrad (°C) | 128 |

Die Gelierzeit bei 160° C beträgt 40 Sekunden.

Beispiel 3

90 Gew.-Teile Diglycidylether des Bisphenol A mit einer Viskosität von 15000 mPa.s/25° C und einem Epoxidäquivalent von 195 werden mit 10 Gew.-Teilen p-Epoxypropoxyphenyl-p-epoxypropoxybenzoat aus Beispiel 1 gemischt. Durch Aufheizen auf 120° C entsteht eine klare Lösung. Die Lösung wird dann auf Raumtemperatur abgekühlt. Auf diese Weise entsteht eine gebrauchsfähige, klare Epoxidharzmischung mit einer Viskosität von 15300 mPa.s/25° C, die nach den üblichen Verfahren kalt und heiß gehärtet werden kann.

## Ansprüche

1. Flüssig-kristalline Diglycidylverbindungen der Formel (I)

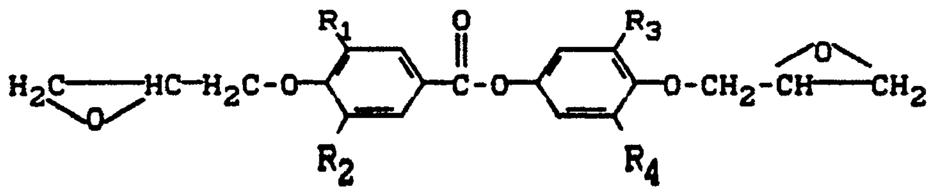

(I)

worin
$R_1$,$R_2$,$R_3$,$R_4$ unabhängig voneinander Wasserstoff, Halogen oder Alkyl mit 1 bis 6 C-Atomen bedeuten.

2. Diglycidylverbindungen nach Anspruch 1, dadurch gekennzeichnet, daß $R_1$, $R_2$, $R_3$, und $R_4$ gleich Wasserstoff ist.

3. Verfahren zur Herstellung von Diglycidylverbindungen der Formel (I) entsprechend Anspruch 1, dadurch gekennzeichnet, daß man gegebenenfalls ringsubstituierte 4-Hydroxyphenyl-4-hydroxybenzoate mit Epichlorhydrin in Gegenwart von Katalysatoren und Alkaliverbindungen, vorzugsweise Natriumhydroxid, in an sich bekannter Weise umsetzt.

4. Härtbare Mischungen, enthaltend Diglycidylverbindungen der Formel (I) nach Anspruch 1, gegebenenfalls in Abmischung mit an sich bekannten Di-, Tri-und Tetraglycidylverbindungen und ein Härtungsmittel für Epoxidharze.

## Claims

1. Liquid-crystalline diglycidyl compounds corresponding to formula (I)

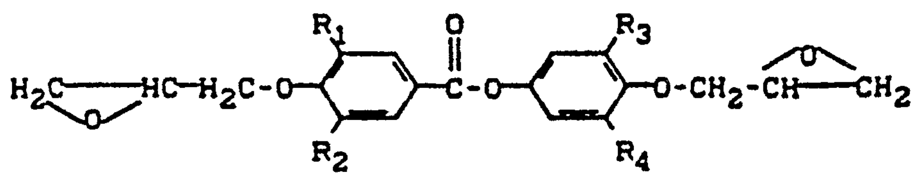

(I)

in which
$R_1$, $R_2$, $R_3$ and $R_4$ independently of one another represent hydrogen, halogen or $C_{1-6}$ alkyl.

2. Diglycidyl compounds as claimed in claim 1, characterized in that $R_1$, $R_2$, $R_3$ and $R_4$ represent hydrogen.

3. A process for the production of the diglycidyl compounds corresponding to formula (I) in claim 1, characterized in that optionally ring-substituted 4-hydroxyphenyl-4-hydroxybenzoates are reacted with epichlorohydrin in the presence of catalysts and alkali compounds, preferably sodium hydroxide, by methods known per se .

4. Curable mixtures containing the diglycidyl compounds corresponding to formula (I) in claim 1, optionally in admixture with di-, tri- and tetraglycidyl compounds known per se and a catalyst for epoxy resins.

## Revendications

1. Composés diglycidyliques à cristaux liquides, de formule (I)

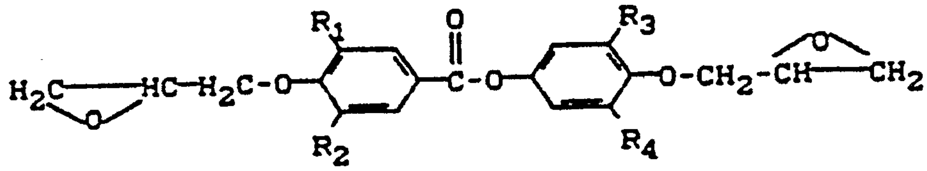 (I)

dans laquelle

$R_1$, $R_2$, $R_3$, $R_4$ représentent, indépendamment les uns des autres, de l'hydrogène, un halogène ou un groupe alkyle ayant 1 à 6 atomes de carbone.

**2.** Composés diglycidyliques suivant la revendication 1, caractérisés en ce que $R_1$, $R_2$, $R_3$ et $R_4$ représentent de l'hydrogène.

**3.** Procédé de production de composés diglycidyliques de formule (I) suivant la revendication 1, caractérisé en ce qu'on fait réagir d'une manière connue des 4-hydroxyphényl-4-hydroxybenzoates éventuellement substitués sur le noyau avec l'épichlorhydrine en présence de catalyseurs et de composés alcalins, de préférence d'hydroxyde de sodium.

**4.** Mélanges durcissables contenant des composés diglycidyliques de formule (I) suivant la revendication 1, éventuellement additionnés de composés diglycidyliques, triglycidyliques et tétraglycidyliques connus et d'un agent durcissant pour résines époxyde.